# EUROPEAN PATENT SPECIFICATION

(11) **EP 4 615 525 B1**
(45) Date of publication and mention of the grant of the patent: **11.11.2026**
(21) Application number: 23793479.9
(22) Date of filing: 11.10.2023
(51) Int. Cl.: A61L 2/00, A61L 2/18, A61L 11/00, A61M 1/00, B08B 3/04

(54) **METHOD FOR OPERATING AN EQUIPMENT FOR PROCESSING ORGANIC SUBSTANCES**
VERFAHREN ZUM BETRIEB EINER ANLAGE ZUR VERARBEITUNG ORGANISCHER SUBSTANZEN
MÉTHODE POUR LE FONCTIONNEMENT D'UN ÉQUIPEMENT POUR LE TRAITEMENT DE SUBSTANCES ORGANIQUES

(30) Priority: 11.11.2022 IT 202200023292
(43) Date of publication of application: 17.09.2025
(73) Proprietor: IN.CAS. S.R.L. - Innovazioni Casamichele, 37062 Villafranca di Verona (VR) (IT)
(72) Inventor: CASAMICHELE, Gianni, 37012 Bussolengo (VR) (IT)
(74) Representative: Ziliotto, Tiziano
(86) International application number: PCT/IB2023/060203
(87) International publication number: WO 2024/100473

(56) References cited:
- US-A1- 2015 362 000

## Description

### TECHNICAL FIELD OF THE INVENTION

The present invention relates to the processing of organic substances, and in particular to the suction operations of potentially infected organic substances. More particularly, the present invention relates to the suction and subsequent disinfection processing of potentially infected organic substances consisting, for example, of the liquid residues of surgical interventions.

### DESCRIPTION OF THE STATE OF THE ART

**In** the hospital environment the need to maintain a hygienically healthy environment in order to prevent the spread of diseases or infections, for example in wards and operating theatres is known.

A particularly felt need concerns the correct processing of all those substances that can be infected, for example all the organic liquids of surgical interventions. These liquids, typically blood and/or urine, are duly collected during the surgical interventions through an appropriate collection bag and then conveyed into appropriate storage containers or temporary storage equipment suitably configured for the disinfection of organic substances, in particular organic liquids.

Such liquids, such as for example urine collected in the hospital wards where bladder washes are performed, can be potentially infected, for example with bacteria carrying more or less dangerous and contagious diseases, such as for example hepatitis B, hepatitis C, HIV, HBV, HCV. The organic liquids must therefore be handled with extreme caution by the operators, to avoid any type of contamination and/or contact, both direct and indirect with the surrounding environment.

Disinfection equipment of known type comprise a main container for the delivery of the organic substance to be processed, a secondary container suited to receive a disinfectant substance and special feeding means suited to convey the disinfectant substance from the secondary container to the main container. The addition of the disinfectant substance accomplishes the desired processing of neutralization or disinfection of the organic substance. The disinfectant substance in order to exert its disinfectant action is used in an appropriate percentage in the organic substance to be processed and acts for an adequate interval of time.

The processed organic substance can then finally be discharged, for example into the sewerage system.

In equipment of known type, the second container for the disinfectant substance consists of a bottle that is connected to a special connector provided in the equipment.

The disinfection equipment of known type is then preferably configured to carry out a self-disinfection cycle which is suitably carried out after the normal use of the equipment for the disinfection of the organic substances.

In the self-disinfection cycle, the internal parts of the equipment, in particular the main container and/or the pipes of the internal hydraulic circuits, are subjected to a cleaning operation using an amount of cleaning liquid, for example water, added with a cleaning and disinfectant substance. This mixture is flowed inside the equipment to hit the parts to be cleaned/disinfected. The cleaning substance is typically in the form of a single-dose capsule which is placed in a dedicated compartment and used during the cleaning cycle.

A first particularly important aspect in the use of disinfection equipment according to the known art is related to the need to guarantee and maintain over time the effectiveness of the disinfection action of the drawn organic substance. Another particularly important aspect is related to the need to ensure the effectiveness of the self-disinfection action of the equipment.

Said aspects are intimately connected to the correct use and management of the disinfectant substance and/or of the cleaning and disinfectant substance used in the respective disinfection and/or self-disinfection cycles US 2015/362000 Al discloses an equipment for processing organic substance and its operation.

An aim of the present invention is to propose a method of operating a disinfection system capable of guaranteeing and maintaining over time the effectiveness of the disinfection action of the drawn organic substance.

Another aim of the present invention is to propose a method of operating a disinfection system capable of guaranteeing and maintaining over time the effectiveness of the self-disinfection action of the equipment.

### SUMMARY OF THE PRESENT INVENTION

The present invention is based on the general consideration that the problems encountered in the state of the art can be at least partially overcome by making an equipment for processing organic substances that comprises a RFID reader. The term "organic substance" below in the text will preferably be understood as a liquid substance. This liquid substance may be more or less dense, as well as contain more or less large-sized suspended particles.

The object of the present invention is a method of operating an equipment for disinfecting an organic substance, as defined in claim 1.

In a first aspect thereof, the present disclosure therefore refers to an equipment for processing an organic substance, said equipment comprising:
- main containment means suited to feed said organic substance to be treated into the inner volume defined by said main containment means;
- an inlet way suited to receive secondary containment means, said secondary containment means being suited to receive a disinfectant substance suited to disinfect said organic substance or suited to receive a cleaning and disinfectant substance suited to disinfect said equipment;
wherein the equipment comprises an RFID reader suited to interact with an RFID tag associated with said secondary containment means.

In a preferred embodiment, the equipment comprises feeding means associated with said inlet way to control the feeding of the disinfectant substance or of the cleaning and disinfectant substance from the secondary containment means into the main containment means.

Preferably the feeding means suited to convey the disinfectant substance comprise a peristaltic pump.

Preferably, the equipment comprises suction means suited to draw said organic substance associated with the feeding inlet.

In a preferred embodiment, the secondary containment means are removably associated with the equipment.

Preferably, the equipment comprises detection means suited to detect the contents inside the main containment means.

According to a preferred embodiment of the disclosure the equipment comprises discharge means associated with the main containment means for discharging the contents of the main containment means.

In a preferred embodiment, the equipment comprises feeding means suited to convey liquid substances and associated with the main containment means.

Preferably, the equipment comprises recirculation means associated with the main containment means for the recirculation of the substances contained in the main containment means.

According to a preferred embodiment of the disclosure the recirculation means cooperate with the suction means suited to draw the organic substance.

Suitably the recirculation means comprise a diffuser element arranged inside the main containment means.

In a preferred embodiment, the equipment comprises a logic control unit connected to the RFID reader.

Preferably the equipment comprises a containment body and wheel means associated with the body.

Advantageously, the equipment comprises a display for displaying and/or entering data.

In another aspect thereof, the present disclosure refers to a unit comprising:
- a disinfectant substance for an organic substance or a cleaning and disinfectant substance for a piece of equipment for processing an organic substance;
- containment means suited to contain said disinfectant substance or said cleaning and disinfectant substance;
wherein said containment means comprises an RFID tag.

According to a preferred embodiment of the disclosure the piece of information of the RFID tag comprises the expiry date and/or the shelf life of the disinfectant substance or the cleaning and disinfectant substance from the opening of the secondary containment means.

Preferably, the RFID tag comprises further information, such as for example the type of disinfectant substance contained, the amount of the contents, the optimal conditions of use, such as for example the temperatures of use, the batch number, a unique identification number.

In a further aspect thereof, the present disclosure refers to a method for operating an equipment as described above wherein the method comprises a step of reading at least one piece of information contained in said RFID tag through said RFID reader, said piece of information being suited to determine whether said disinfectant substance or said cleaning and disinfectant substance has expired.

In a preferred embodiment, the method comprises a step of inhibiting the feeding of the disinfectant substance or of the cleaning and disinfectant substance into the main containment means if said disinfectant substance or said cleaning and disinfectant substance has expired.

Preferably, the method comprises a step of generating a signal suited to indicate that the disinfectant substance or the cleaning and disinfectant substance has expired.

According to a preferred embodiment of the disclosure the piece of information of the RFID tag read comprises the expiry date and/or the shelf life of the disinfectant substance or the cleaning and disinfectant substance from the opening of the secondary containment means.

### BRIEF DESCRIPTION OF THE DRAWINGS

Further advantages, objects and features, as well as embodiments of the present invention are defined in the claims and will be clarified hereinafter by means of the following description, in which reference is made to the accompanying drawing tables; in the drawings, corresponding or equivalent features and/or component parts of the present invention are identified by the same reference numerals. In particular:
- Figure 1 represents in axonometric view an equipment according to a preferred embodiment of the disclosure
- Figure 2 represents the functional diagram of the equipment of Figure 1 according to a preferred embodiment of the disclosure
- Figure 3 represents operation steps of the equipment of Figure 1 with reference to the functional diagram of Figure 2;
- Figure 4 represents the functional diagram of the equipment of Figure 1 according to a further preferred embodiment of the disclosure
- Figures 5 to 6 represent different operation steps of the equipment of Figure 1 with reference to the functional diagram of Figure 4;
- Figure 7 represents the functional diagram of the equipment of Figure 1 according to another preferred embodiment of the disclosure
- Figure 8 represents operation steps of the equipment of Figure 1 with reference to the functional diagram of Figure 7.

### DETAILED DESCRIPTION OF PREFERRED EMBODIMENTS OF THE PRESENT DISCLOSURE

The present invention finds particular, but not exclusive, application in the hospital environment. In particular, the equipment for processing organic substances of the disclosure can be advantageously used during the surgical interventions. The term "organic substance" will therefore preferably be understood as a liquid substance, such as for example blood or urine. This liquid substance may be more or less dense, as well as contain more or less large-sized suspended particles of other type.

For this reason, the present disclosure will be presented below with particular reference to its application during a surgical intervention. It should however be noted that the applications of the present disclosure are not limited to the case of surgical interventions; on the contrary, the present disclosure also finds an advantageous application in all those cases in which it is necessary to process organic substances, preferably liquids, potentially infected. The equipment of the disclosure may find advantageous application, for example, in dental surgeries, in outpatient clinics, in nursing homes, etc.

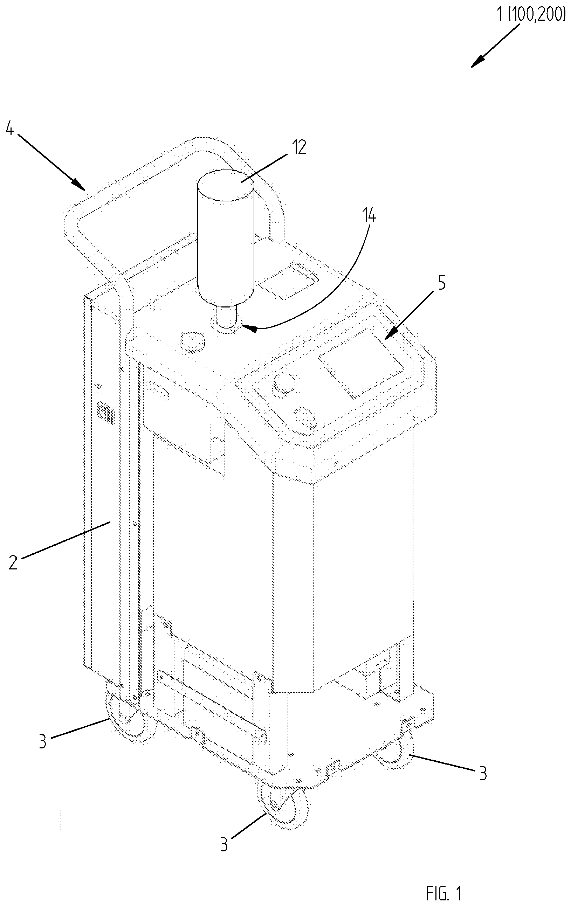

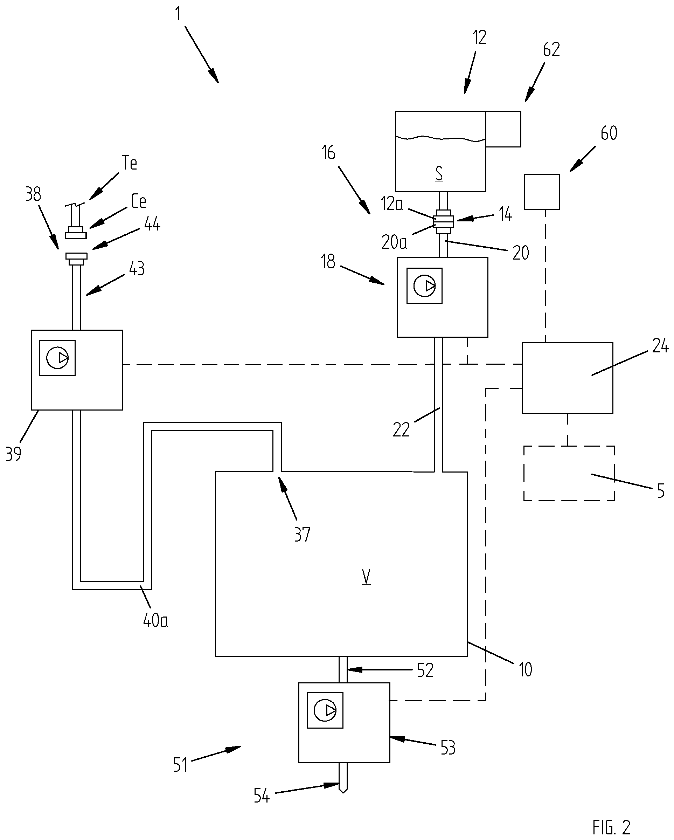

In the following, with reference to Figures 1 and 2, a first embodiment of the equipment 1 for processing organic substances will be described.

For simplicity of presentation, in the continuation of the present description the equipment for disinfecting organic substances 1 of the disclosure will be simply referred to as equipment 1.

A preferred embodiment of the equipment 1 according to the present disclosure is shown in Figure 1.

The equipment 1 is made according to a movable configuration and comprises a body 2, preferably made of ABS (Acrylonitrile Butadiene Styrene), mounted on wheels 3. This makes the equipment 1 particularly shock-resistant. The wheels 3 are preferably pivoting and antistatic, as well as provided with brakes. In this way, the equipment 1 is easily transportable and protected against any electrostatic discharges. An external manoeuvring handle 4 facilitates its movement.

A display 5, preferably of the touch-screen type, allows the operator to enter and display the various operating data, as we will see better below.

The aforesaid characteristics, together with others described below, make the use of equipment 1 particularly suitable within hospital environments, such as wards or operating rooms.

With reference to the schematic representation of Figure 2, the equipment 1 of Figure 1 is described in detail.

The equipment 1 comprises main containment means 10, or main container 10, suited to define an inner volume V for receiving the organic substance O to be processed.

The term "organic substance" O is intended to mean, in the continuation of the description, any type of substance, preferably liquid, which may comprise elements of risk, such as bacteria carrying infectious diseases such as hepatitis B, hepatitis C, HIV, HBV, HCV, etc.

In a preferred embodiment, in the equipment according to the disclosure the organic substance O received in the main container 10 is also subjected to a processing to neutralize said risk elements.

Such neutralization or disinfection processing advantageously consists in the disinfection of the organic substance by adding a special disinfectant substance S. The disinfectant substance S in order to exert its disinfecting action should preferably be present in an adequate percentage in the organic substance O to be processed and act for a predetermined time interval. This time interval must be greater than a minimum time required to allow an effective disinfectant action.

In one aspect of the present disclosure the disinfectant substance S is advantageously received in a secondary container 12. The secondary container 12 is removably associated with the equipment 1. For this purpose, the equipment 1 comprises an inlet way 14 suited to receive the secondary container 12.

In a preferred embodiment, the secondary container 12 consists of a bottle, as shown in Figure 1.

At the inlet way 14 there are associated feeding means 16 suited to control the feeding of the disinfectant substance S from the secondary container 12 to the main containment 10.

Preferably such feeding means 16 comprise a peristaltic pump 18, a first withdrawal duct 20 suited to withdraw from the secondary container 12 and a second conveying duct 22 to the main container 10.

The first duct 20 is provided with an end connector 20a connectable to a corresponding connector 12a of the secondary container 12.

A logic control unit 24, preferably comprising an electronic control board, suitably controls the peristaltic pump 18.

The controlled operation of the peristaltic pump 18 allows the withdrawal of doses of the disinfectant substance S from the secondary container 12 and their introduction into the main container 10.

In embodiment variants of the disclosure such feeding means may be of a different type, but suitable for the purpose, such as for example other types of pump.

A first feeding inlet 37 suited to feed the organic substance O to be processed is associated with the main container 10. For this purpose, the equipment 1 comprises an inlet circuit 38 for the introduction of the organic substance O, collected externally, into the main container 10.

The inlet circuit 38 preferably comprises a suction pump 39 connected to the first inlet 37 via a first way 40a. The suction pump 39 is suitably connected to the logic control unit 24.

A suction pipe 43 provided with an end connector 44 is connected to the suction pump 39.

During operation of the equipment 1, an outer suction duct Te will be suitably connected to the end connector 44, as we will see better below.

A discharge circuit 51 is associated with the main container 10. The discharge circuit 51 preferably comprises a withdrawal duct 52 arranged at the bottom of the container 10, discharge means 53, preferably a discharge pump, and an outlet duct 54.

The discharge means 53 are advantageously connected to the logic control unit 24.

The outlet duct 54 can advantageously be connected, if necessary, to a discharge channel, such as for example the sewerage system.

Display and data entry means 5, preferably in the form of a display, already indicated above with reference to Figure 1, are advantageously connected to the logic control unit 24.

According to one aspect of the present disclosure the equipment 1 comprises an RFID reader 60.

The RFID reader 60 is preferably positioned in proximity to the inlet way 14 so as to be as close as possible to the secondary container 12.

The RFID reader 60 is advantageously connected to the logic control unit 24. According to another aspect of the present disclosure the secondary container 12 comprises an RFID tag 62.

The RFID reader 60 and the RFID tag 62 allow the handling of the disinfectant substance S, as better described below.

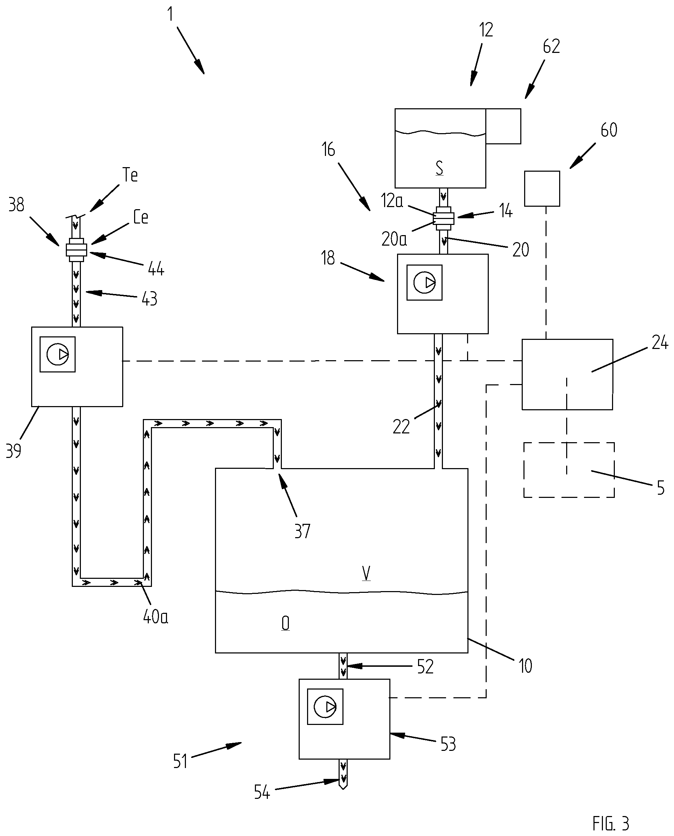

With reference to Figure 3, the possible operation of the equipment 1 of Figure 2 according to a preferred embodiment of the disclosure is described below.

The use of the equipment 1 provides, first of all, for the connection of its inlet circuit 38 to the collection point of the organic substance O to be processed. In particular, the end connector 44 of the suction pipe 43 is connected to a corresponding connector Ce of the outer suction pipe Te, as indicated in the figure.

The outer suction tube Te may be associated, for example, with a liquid collection bag used in the surgical interventions.

Once the connection is made with the outer suction pipe Te, the equipment 1 is ready for operation.

During such operation, the organic substance O is drawn through the inlet circuit 38 and introduced into the main container 10.

The organic substance O reaches the main container 10 from the first inlet 37, as schematically indicated by the arrows.

The control of the suction pump 39 is advantageously realized by the logic control unit 24.

The actuation of the suction pump 39 can be of a continuous type, in the case of high qualities of organic substance O coming from the outer suction pipe Te, or of an intermittent type, or again with a controlled actuation on the basis of the flow coming from the outer pipe Te. In the latter case, it may be provided for an appropriate flow sensor associated with the inlet circuit 38.

Advantageously, the suction speed of the suction pump 39 can be chosen by the operator through, for example, the display 5, where an appropriate page for setting the type of operation will be displayed, or selectable for that purpose. The organic substance O is therefore introduced into the inner volume V of the main container 10.

The logic control unit 24 then acts on the feeding means 16 suited to convey the disinfectant substance S.

The logic control unit 24 drives the peristaltic pump 18 for introducing a dose of disinfectant substance S into the main container 10. Preferably, said dose of disinfectant substance S is introduced on the basis of the amount of organic substance O present in the main container 10. In particular, a dose D of disinfectant substance S is introduced into the main container 10 whenever the organic substance O inside the main container 10 increases by a predetermined amount Q. For example, a dose D of 5 cc is added at each increase in organic substance O by an amount Q equal to 500 cc.

The increase in organic substance O of said quantity Q can be easily calculated by the logic control unit 24 by processing values detected by suitable sensor means suited to provide the filling level of the main container 10, for example a float with a magnetoresistive sensor, as described below. The dose D of disinfectant substance S can be easily made available, for example, through a predetermined number of revolutions of the peristaltic pump 18.

Inside the main container 10 the amount of disinfectant substance S is preferably maintained in a controlled percentage ratio R with respect to the organic substance O, for example of 1% in the case indicated above. It is evident that in embodiment variants this percentage ratio R may be different. This percentage ratio R may depend, for example, on the type of disinfectant substance S used. Preferably, this percentage ratio value R can be chosen by the operator through, for example, the display 5, where an appropriate page for setting this percentage ratio will be displayed, or selectable for that purpose.

In a preferred embodiment of the disclosure the capacities of the main container 10 and of the secondary container 12 are chosen on the basis of this percentage value.

The percentage ratio R between disinfectant substance S and organic substance O represents, as mentioned, a very important parameter for the correct performance of the disinfection process. In general, the equipment shall ensure that this percentage ratio R is greater than or equal to a predetermined minimum percentage ratio Rmin.

In one embodiment, for example if there are no feeding means associated with the secondary container such as the peristaltic pump 18, the disinfectant substance S is introduced directly from the secondary container 12 into the main container 10 according to the predetermined amount present therein.

In addition to what has been said with reference to the parameter consisting of the percentage ratio R of disinfectant substance S with respect to the organic substance O, another essential control parameter on the effectiveness of the disinfectant action is the time of action ta of the disinfectant substance S in the organic substance O.

The time of action ta is monitored for this purpose by the control unit 24 which will signal, for example by means of a message on the display 5, when this time of action ta has elapsed.

In addition, special safety systems may be provided to prevent access to the organic substance O before this time of action has elapsed. For example, an acoustic and/or visual signalling may be provided.

Once the time of action ta has elapsed, the disinfected organic substance mixture O' will be present inside the main container 10.

The equipment 1 is therefore ready for the discharge of this disinfected organic substance O'. This may preferably occur immediately or, alternatively, at a later time. Preferably, said discharge operation takes place in the full load condition of the main container 10, for example detected through said sensor.

The secondary container 12 will advantageously be empty, as mentioned above. In embodiment variants of the disclosure the discharge operation can be carried out with any amount of disinfected organic substance O' present in the main container 10, in this case taking care to ensure that the disinfectant substance S and the organic substance O are in the right percentage ratio R and that the time of action ta has elapsed.

The discharge step takes place by means of the discharge circuit 51. The discharge pump 53 is driven and the disinfected organic substance O is collected via the withdrawal duct 52 and conveyed to the outlet duct 54, as schematically indicated by the arrows in Figure 3.

Advantageously, the outlet duct 54 can be connected directly to the sewerage system.

At this point, the discharge step can be considered as concluded.

With reference to what has been described, it should be noted that the effectiveness of the disinfection of the organic substance O is clearly linked to the effectiveness of the disinfectant substance S in the secondary container 12 which is placed in the main container 10.

In fact, the disinfectant substances S used have a limited duration in time and are therefore characterized by an expiry date. The disinfectant substances S can in fact be characterised by a final expiry date beyond which the effectiveness of disinfection is no longer guaranteed or they can be characterised by a shelf life starting from the opening of the packaging/container that contains them.

According to one aspect of the present disclosure the equipment 1 allows to manage the disinfectant substance S present in the secondary container 12.

As mentioned above, an RFID tag 62 is associated with the secondary container 12. The RFID tag 62 contains, in particular, information useful for identifying the shelf life of the disinfectant substance S inside the secondary container 12.

In particular, the RFID tag 62 may contain the expiry date Ds of the disinfectant substance S and/or the shelf life Pv starting from the opening of the secondary container 12.

Additional information may then be associated with the RFID tag 62, such as for example the type of disinfectant substance S contained, the amount of the contents, the optimal conditions of use, such as for example the temperatures of use, the batch number, a unique identification number, etc.

Such information is preferably associated with the secondary container 12 during the step of filling the secondary container 12 with the disinfectant substance S by the manufacturer. More preferably, such information is stored in a suitable memory area of the electronic unit (microchip) of the RFID tag 62.

At the moment in which the secondary container 12 is applied or brought closer to the equipment 1, preferably by connecting the connector 12a of the secondary container 12 to the end connector 20a of the first duct 20, the RFID reader 60 advantageously has access to the information of the RFID tag 62 of the secondary container 12. This information is acquired and appropriately used by the logic control unit 24.

By way of example, the logic control unit 24 acquires the expiry date of the disinfectant substance S and provides for its use in disinfection cycles, for example according to the methodology described above, only if it has not expired on the date of use. Otherwise, if the date of use is later than the expiry date, the equipment 1 prevents its use and preferably proceeds to signal the expiration to the user through an appropriate message, for example a visual signalling through the display 5 and/or a special acoustic signal.

On the basis of said information, the logic control unit 24 will then be able to generate further messages for the user, for example the indication of the days until the expiry date.

**In** another embodiment, the logic control unit 24 establishes the date of opening of the secondary container 12 when it is applied to the equipment 1. **In** this case, if the RFID reader 60 acquires from the RFID label 62 the information that the disinfectant substance is characterized by a shelf life Pv starting from the opening of the secondary container 12, the logic control unit 24 will allow the use of the disinfectant substance S only within the shelf life and will preferably proceed to signal to the user the possible expiration, for example by means of a visual signalling through the display 5 and/or a special acoustic signal.

Preferably, in this case, the secondary container 12 is used by a single equipment 1 capable of recognizing that particular secondary container 12 and associating the previously determined opening date.

In a preferred embodiment, the RFID reader 60 is further configured to write data on the RFID tag 62.

Preferably, the RFID reader 60 is configured to write on the RFID tag 62 the date of opening of the secondary container 12 in a special memory area of the RFID tag 62. In this case, preferably, the secondary container 12 can also be used in different equipment equipped with an RFID reader 60 capable of reading and detecting the opening date of the secondary container 12 and capable of consequently managing its use before the actual expiration.

At the end of the discharge step, as described above, the equipment 1 is suitably subjected to a disinfection step. This step is intended to carry out the cleaning and the disinfection of the main container 10 and of the inner circuits of the equipment 1 that have come into contact with the organic substance O.

The equipment 1 and the various parts that constitute it and that need powering are preferably powered by low-voltage batteries, for example 24V, and advantageously of a rechargeable type. This makes it safe to use, especially inside an operating room.

In a preferred embodiment, the discharge means are instead powered by the mains, i.e. with 220V. In this way, the use of a discharge pump of sufficiently high power, for example 37 litres/minute, can be envisaged in order to make the discharge step as fast as possible.

For what is described above, it is clear how the equipment 1 allows to achieve the preset purposes.

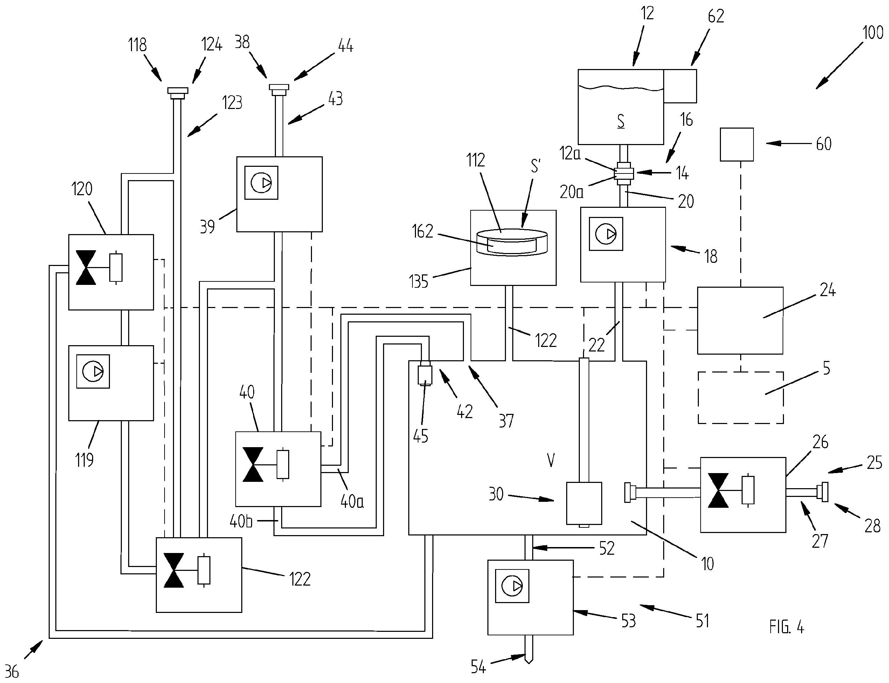

With reference to the schematic representation of Figure 4, an embodiment variant of an equipment 100 according to the disclosure is described; features and/or component parts similar or equivalent to the previously described embodiment are identified by the same reference numerals.

Parts of the equipment 100 that differ from the equipment 1 previously described and illustrated in Figure 2 will be described below more specifically.

The equipment 100 comprises a main container 10 and a secondary container 12 suited to receive the disinfectant substance S, preferably configured as described above.

A first feeding inlet 37 suited to feed the organic substance O to be processed is associated with the main container 10. For this purpose, the equipment 100 comprises a first inlet circuit 38 and a second inlet circuit 118 for the introduction of the organic substance O, collected externally, into the main container 10.

The first inlet circuit 38 preferably comprises a first suction pump 39 connected to the first inlet 37 of the main container 10 by means of controlled first opening/closing means 40. Such first controlled opening/closing means 40 preferably comprise a three-way solenoid valve 40 with two outlet ways 40a, 40b. The first outlet way 40a is connected to said first inlet 37 of the main container 10. The second outlet way 40b is connected to a second inlet 42 of the main container 10. Preferably said second inlet 42 comprises a diffuser element 45, such as for example a 360° rotating nozzle.

Both the first suction pump 39 and the first solenoid valve 40 are suitably connected to the logic control unit 24.

A first suction pipe 43 provided with a first end connector 44 is connected to the first suction pump 39.

The second inlet circuit 118 preferably comprises a second suction pump 119, second controlled opening/closing means 120 and third controlled opening/closing means 122. The second controlled opening/closing means 120 preferably comprise a second three-way solenoid valve 120. The third controlled opening/closing means 122 preferably comprise a third three-way solenoid valve 122. The second suction pump 119 and the solenoid valves 120, 122 are suitably connected to the logic control unit 24.

A second common suction pipe 123 provided with a second end connector 124 is connected to the second and third solenoid valve 120, 122.

At the bottom of the main container 10 there is associated a recirculation duct 36 connected at the inlet to the second solenoid valve 120.

During operation of the equipment 100, as described below, corresponding outer suction ducts Te1, Te2 will be suitably connected to the end connectors 44, 124.

The equipment 100 then comprises a liquid feeding circuit suited to feed liquid substances 25. Said feeding circuit suited to feed liquid substances 25 preferably comprises controlled opening/closing means 26, for example a solenoid valve, and a respective inlet duct 27 provided with an end connector 28.

The opening/closing means 26 are suitably connected to the logic control unit 24. Preferably, said feeding circuit suited to feed liquid substances 25 is connected via the end connector 28 to a hydraulic water feeding circuit.

Detection means suited to detect its filling level 30 are associated with the main container 10. In the embodiment shown, the detection means 30 are arranged inside the main container 10 and consist of one or two floats with magnetoresistive sensor suited to slide vertically depending on the level of the amount of substance present inside the container 10. The detection means 30 advantageously provide the filling value of the main container 10 to the logic control unit 24.

In embodiment variants, the detection means suited to detect the contents of the main container may be of a different type, such as for example optical type detection means, or flow sensors, etc.

The main container 10 is associated with a second inlet way 135, preferably consisting of a seat 135, communicating with the inside of the main container 10, suited to receive a cleaning and disinfectant substance S' of the equipment 100. Advantageously the cleaning and disinfectant substance S' is in the form of a single-dose capsule 112. The single-dose capsule 112 defines the container for the cleaning and disinfectant substance S'.

According to one aspect of the present disclosure the container/capsule 112 comprises an RFID tag 162.

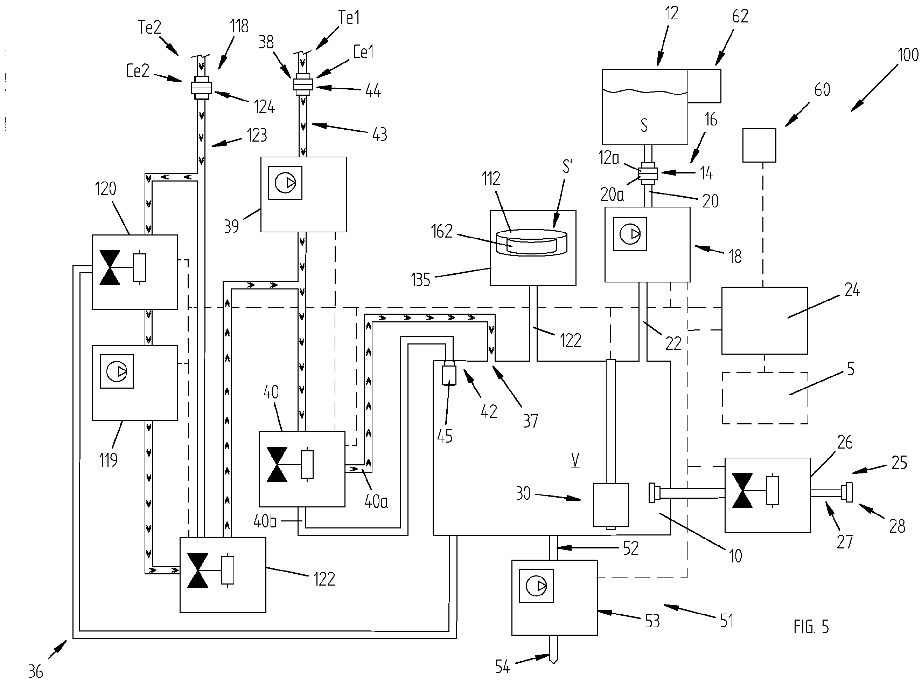

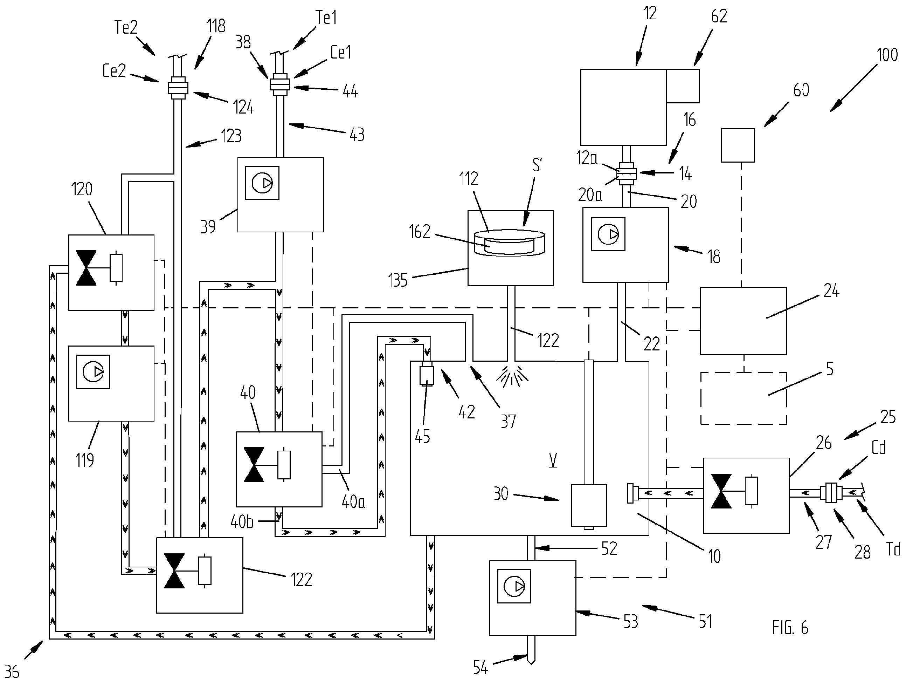

With reference to Figures 5 and 6, the operation of the equipment 100 of Figure 4 according to a preferred embodiment of the disclosure is described below.

The equipment 100 provides for the connection of the first inlet circuit 38 and of the second inlet circuit 118 to respective two collection points of the organic substance O to be processed. In particular, the first end connector 44 of the first suction pipe 43 is connected to a corresponding first connector Ce1 of a first outer suction pipe Te1, as illustrated in Figure 5. The second end connector 124 of the second suction pipe 123 is connected to a corresponding second connector Ce2 of a second outer suction pipe Te2.

The first or second outer suction tube Te1, Te2 may be associated, for example, with respective liquid collection bags used in the surgical interventions.

Once the connection is made with the outer suction pipes Te1, Te2, the equipment 100 is ready for operation.

During such operation, the organic substance O is drawn through the first inlet circuit 38 and through the second inlet circuit 118 and introduced into the main container 10.

For this purpose, the first three-way solenoid valve 40 is suitably controlled to convey the organic substance O drawn through the first outlet 40a. The organic substance O reaches the main container 10 from the first inlet 37, as schematically indicated by the path of the arrows along the ducts.

At the same time, the first suction pump 39 and the second suction pump 119 are suitably controlled to convey the organic substance O coming from the outer suction pipes Te1, Te2 to the first three-way solenoid valve 40.

In particular, the second and third solenoid valves 120, 122 are suitably controlled, according to the configuration shown in Figure 5, to convey the organic substance O drawn from the second suction pump 119 and coming from the second outer suction pipe Te2 towards the first three-way solenoid valve 40. The control of the suction pumps 39, 119 and of the three-way solenoid valves 40, 120, 122 is advantageously realized by the logic control unit 24.

In the preferred operation mode described above, the suction of the organic substance O takes place through both the inlet circuits 38, 118. In an embodiment variant, the suction step can be carried out by only one of the two inlet circuits 38, 118, in this case by appropriately controlling the suction pumps 39, 119 and the three-way solenoid valves 40, 120, 122.

The remaining operation steps of the equipment 100 may preferably coincide with the steps described above with reference to the equipment of Figure 2, in particular with reference to the management of the introduction of disinfectant substance S inside the main container 10.

With reference to Figure 6, the configuration of the equipment 100 with regard to the disinfection step, or self-disinfection, of the equipment 100 is described below. As mentioned above, this step is intended to carry out the cleaning and the disinfection of the main container 10 and of the inner circuits of the equipment 100 that have come into contact with the organic substance O.

The disinfection step envisages using a cleaning and disinfectant substance S', preferably the cleaning and disinfectant substance S' in the form of a capsule 112 positioned inside the appropriate reception site 135.

The cleaning and disinfectant substance S' is then introduced into the main container 10, which will be substantially empty following the discharge step previously carried out.

An amount of cleaning liquid L, advantageously water, is then introduced into the main container 10 via the liquid feeding circuit 25. For this purpose, the end connector 28 of the inlet duct 27 is advantageously connected to a feeding pipe Td of the cleaning liquid L through a respective connector Cd.

The amount of cleaning liquid L introduced will be of such value as to guarantee the percentage ratio required for the particular cleaning and disinfectant substance S' used, for example 15lt of cleaning liquid L for said single dose of cleaning and disinfectant substance S' equal to 20cc.

At the same time, the second suction pump 119 and the three three-way solenoid valves 40, 120, 122 are suitably controlled to obtain the configuration shown in the figure.

**In** this configuration, a recirculation circuit is created which, schematically indicated with the arrows, starts from the bottom of the main container 10, follows the recirculation duct 36, passes through the second solenoid valve 120, the second suction pump 119, the third solenoid valve 122, the first solenoid valve 40 and then through the second way 40b of the solenoid valve 40 to reach the second inlet 42 of the main container 10.

The recirculation circuit is therefore put into operation by driving the second suction pump 119.

The disinfectant mixture inside the main container 10, consisting of cleaning liquid L and of the cleaning and disinfectant substance S', is therefore made to flow inside the recirculation circuit and sprayed inside the main container 10 through the nozzle 45 at 360°.

The nozzle 45 advantageously directs the disinfectant mixture onto all inner surfaces of the main container 10.

Furthermore, by suitably driving the first solenoid valve 40 towards the first way 40a it is possible to recirculate the disinfectant mixture also through the pipe that connects the first inlet 37 of the main container 10.

At the end of this operation, a discharge operation follows by driving the discharge means 51 for the evacuation of the disinfectant mixture.

The equipment 100 will then be ready for further use.

With reference to what has been described, it should be noted that the effectiveness of the self-disinfection phase of the equipment is clearly linked to the effectiveness of the cleaning and disinfection substance S' in the capsule 112 which is introduced into the main container 10.

Similar to what was said above for the disinfectant substances S, the cleaning and disinfectant substances S' also have a limited duration in time and are therefore characterized by an expiry date. The cleaning and disinfectant substances S' can in fact be characterized by a final expiry date beyond which the effectiveness of disinfection is no longer guaranteed or they can be characterized by a shelf life starting from the opening of the package/container/capsule that contains them. According to one aspect of the present disclosure the equipment 100 allows to manage the use of the cleaning and disinfectant substance S' present in the capsule 112.

As mentioned above, an RFID tag 162 is associated with the capsule 112. The RFID tag 162 contains, in particular, information useful for identifying the shelf life of the cleaning and disinfectant substance S' inside the capsule 112.

In particular, the RFID tag 162 may contain the expiry date Ds of the disinfectant substance S and/or the shelf life Pv starting from the opening of the capsule 112.

Additional information may then be associated with the RFID tag 162, such as for example the type of cleaning and disinfectant substance S' contained, the amount of the contents, the optimal conditions of use, such as for example the temperatures of use, the batch number, a unique identification number, etc.

Such information is preferably associated with the capsule 112 during the step of filling the capsule 112 with the cleaning and disinfectant substance S' by the manufacturer. More preferably, such information is stored in a suitable memory area of the electronic unit (microchip) of the RFID tag 162.

At the time in which the capsule 112 is inserted into the seat 135 of the equipment 100 the RFID reader 60 advantageously has access to the information of the RFID tag 162 of the capsule 112. This information is acquired and appropriately used by the logic control unit 24.

By way of example, the logic control unit 24 acquires the expiry date of the cleaning and disinfection substance S' and provides for its use in disinfection cycles, for example according to the methodology described above, only if it is not expired on the date of use. Otherwise, if the date of use is later than the expiry date, the equipment 100 prevents its use and preferably proceeds to signal the expiration to the user through an appropriate message, for example a visual signalling through the display 5 and/or a special acoustic signal.

On the basis of said information, the logic control unit 24 will then be able to generate further messages for the user, for example the indication of the days until the expiry date.

In another embodiment, the logic control unit 24 establishes the date of opening of the capsule 112 when it is inserted into the seat 135 of the equipment 100. In this case, if the RFID reader 60 acquires from the RFID label 162 the information that the cleaning and disinfectant substance is characterized by a shelf life Pv starting from the opening of the capsule 112, the logic control unit 24 will allow the use of the cleaning and disinfectant substance S' only within the shelf life and will preferably proceed to signal to the user the possible expiration, for example by means of a visual signalling through the display 5 and/or a special acoustic signal.

Preferably, in such a case, the capsule 112 is used by a single equipment 100 capable of recognizing that particular capsule 112 and associating the previously determined opening date.

In a preferred embodiment, the RFID reader 60 is further configured to write data on the RFID tag 162.

Preferably, the RFID reader 60 is configured to write the opening date of the capsule 112 on the RFID tag 162 in a dedicated memory area of the RFID tag 162. In this case, preferably, the capsule 112 can also be used in different equipment equipped with an RFID reader 60 capable of reading and detecting the opening date of the capsule 112 and capable of consequently managing its use before the actual expiry.

From what is described above, it is clear how the equipment 100 allows to achieve the intended purposes with regard to the management of the disinfectant substance S' and/or the cleaning and disinfectant substance S'.

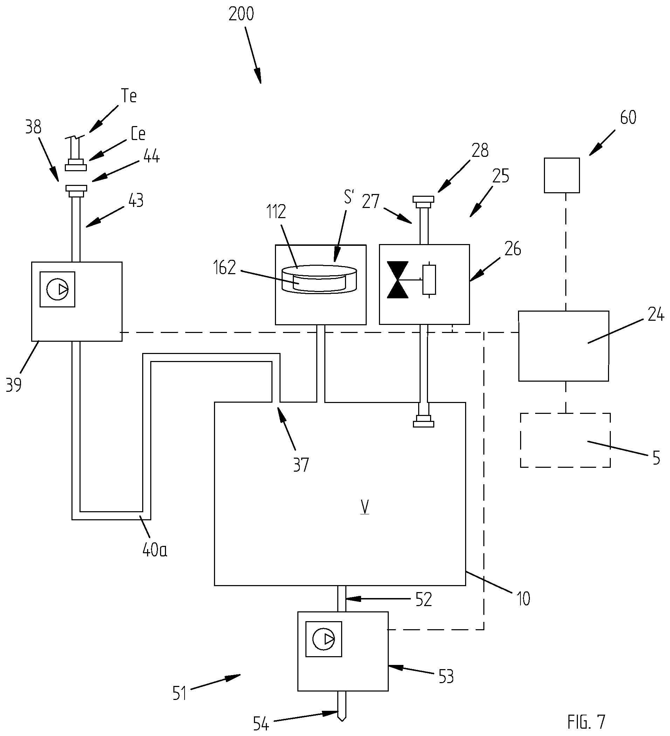

With reference to the schematic representation of Figure 7, an embodiment variant of an equipment 200 according to the disclosure is described; features and/or component parts similar or equivalent to the previously described embodiments are identified by the same reference numerals.

The equipment 200 comprises main containment means 10, or main container 10, suited to define an inner volume V for receiving the organic substance O to be processed.

A first feeding inlet 37 suited to feed the organic substance O to be processed is associated with the main container 10. For this purpose, the equipment 200 comprises an inlet circuit 38 for the introduction of the organic substance O, collected externally, into the main container 10.

The inlet circuit 38 preferably comprises a suction pump 39 connected to the first inlet 37 via a first way 40a. A logic control unit 16, preferably comprising an electronic control board, suitably controls the suction pump 39. A suction pipe 43 provided with an end connector 44 is connected to the suction pump 39.

During operation of the equipment 200, an external suction duct Te is suitably connected to the end connector 24, as described below.

A discharge circuit 51 is associated with the main container 10. The discharge circuit 51 preferably comprises a withdrawal duct 52 arranged at the bottom of the container 10, discharge means 53, preferably a discharge pump, and an outlet duct 54.

The discharge means 53 are advantageously connected to the logic control unit 16.

The outlet duct 54 can advantageously be connected, if necessary, to a discharge channel, such as for example the sewerage system.

Display and data entry means 5, preferably in the form of a display, already indicated above with reference to Figure 1, are advantageously connected to the logic control unit 16.

The equipment 200 then comprises a feeding circuit suited to feed liquid substances 25. Said feeding circuit suited to feed liquid substances 25 preferably comprises controlled opening/closing means 26, for example a solenoid valve, and a respective inlet duct 27 provided with an end connector 28.

The opening/closing means 26 are suitably connected to the logic control unit 24. Preferably, said feeding circuit suited to feed liquid substances 25 is connected via the end connector 28 to a hydraulic water feeding circuit.

The main container 10 is associated with an inlet way 135, preferably consisting of a seat 135, communicating with the inside of the main container 10, suited to receive a cleaning and disinfectant substance S' of the equipment 200. Advantageously the cleaning and disinfectant substance S' is in the form of a single-dose capsule 112. The single-dose capsule 112 defines the container for the cleaning and disinfectant substance S'.

According to one aspect of the present disclosure the container/capsule 112 comprises an RFID tag 162.

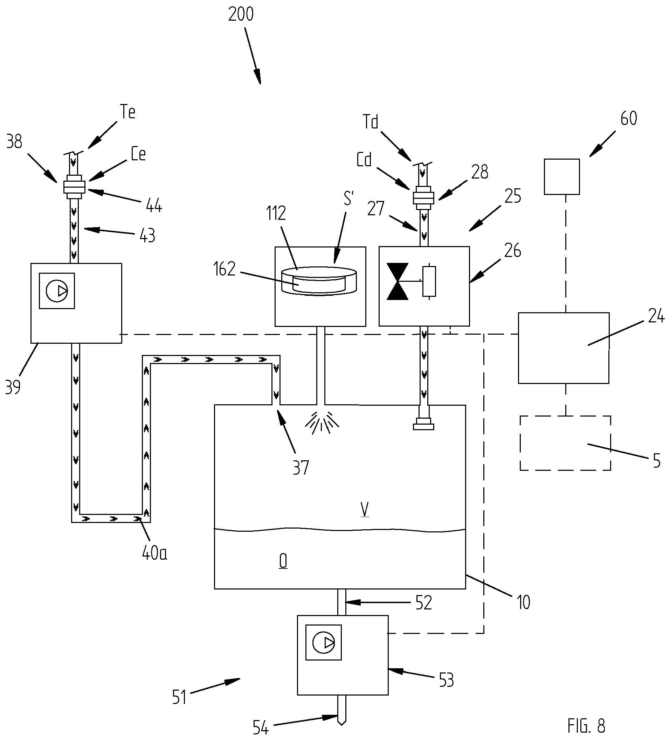

With reference to Figure 8, the possible operation of the equipment 200 of Figure 7 according to a preferred embodiment of the disclosure is described below.

The use of the equipment 200 provides, first of all, for the connection of its inlet circuit 38 to the collection point of the organic substance O. In particular, the end connector 44 of the suction pipe 43 is connected to a corresponding connector Ce of the outer suction pipe Te.

The outer suction tube Te may be associated, for example, with a liquid collection bag used in the surgical interventions.

Once the connection is made with the outer suction pipe Te, the equipment 200 is ready for operation.

During such operation, the organic substance O is drawn through the inlet circuit 38 and introduced into the main container 10.

The organic substance O reaches the main container 10 from the first inlet 37, as schematically indicated by the arrows.

The control of the suction pump 39 is advantageously realized by the logic control unit 24.

The actuation of the suction pump 39 can be of a continuous type, in the case of high qualities of organic substance O coming from the outer suction pipe Te, or of an intermittent type, or again with a controlled actuation on the basis of the flow coming from the outer pipe Te. In the latter case, it may be provided for an appropriate flow sensor associated with the inlet circuit 38.

Advantageously, the suction speed of the suction pump 39 can be chosen by the operator through, for example, the display 5, where an appropriate page for setting the type of operation will be displayed, or selectable for that purpose. The organic substance O is therefore introduced into the inner volume V of the main container 10.

The equipment 200 with the drawn organic substance O therefore acts as a reservoir, avoiding any contact with the operators.

The organic substance O may be subsequently discharged. Preferably, said discharge operation takes place in the full load condition of the main container 10, for example detected through said sensor.

The discharge step takes place by means of the discharge circuit 51. The discharge pump 53 is driven and the organic substance O is collected via the withdrawal duct 52 and conveyed to the outlet duct 54, as schematically indicated by the arrows in the figure.

Advantageously, the outlet duct 54 can be connected directly to the sewerage system.

At this point the processing of the organic substance O can be considered as concluded. The processing of the organic substance O substantially corresponds to the suction and temporary storage in an airtight volume inside the equipment 200 until the next discharge.

At the end of the discharge step, the equipment 200 is suitably subjected to a disinfection, or self-disinfection step.

The disinfection step envisages using the cleaning and disinfectant substance S', preferably the cleaning and disinfectant substance S' in the form of a capsule 112 positioned inside the appropriate reception site 135.

The cleaning and disinfectant substance S' is then introduced into the main container 10, which will be substantially empty following the discharge step previously carried out.

An amount of cleaning liquid L, advantageously water, is then introduced into the main container 10 via the liquid feeding circuit 25. For this purpose, the end connector 28 of the inlet duct 27 is advantageously connected to a feeding pipe Td of the cleaning liquid L through a respective connector Cd.

The amount of cleaning liquid L introduced will be of such value as to guarantee the percentage ratio required for the particular cleaning and disinfectant substance S' used, for example 15lt of cleaning liquid L for said single dose of cleaning and disinfectant substance S' equal to 20cc.

The disinfectant mixture inside the main container 10, consisting of the cleaning liquid L and the cleaning and disinfectant substance S', therefore affects the inner surfaces of the main container 10.

In a preferred embodiment, diffusion means, for example a spray diffuser element, will be provided inside the main container, which are suited to allow the conveying of the disinfectant mixture on all the inner surfaces of the main container.

At the end of this operation, a discharge operation follows by driving the discharge means 51 for the evacuation of the disinfectant mixture.

The equipment 200 will then be ready for further use.

In embodiments, not illustrated, the equipment may be provided with further ducts and/or controlled opening/closing means (solenoid valves) to allow the disinfection of the various ducts hit by the organic substance O.

According to one aspect of the present disclosure and as described above, the equipment 200 allows to manage the use of the cleaning and disinfectant substance S' present in the capsule 112 thanks to the presence of the RFID reader 60 and the RFID tag 162 associated with the capsule 112.

In particular, the use of cleaning and disinfectant substance S' before its expiration is guaranteed.

It has therefore been shown that the present disclosure presented above allows the intended purposes to be achieved.

In particular, the present invention allows to correctly manage the use of the disinfectant substance and/or the cleaning and disinfectant substance used in the respective disinfection and/or self-disinfection cycles.

While the present invention has been described with reference to the particular embodiments represented in the figures, it should be noted that the present invention is not limited to the particular embodiments represented and described; on the contrary, further variants of the described embodiments fall within the scope of the present invention, which scope is defined by the claims.

## Claims

1. Method for operating an equipment (1; 100; 200) for processing an organic substance (O), said equipment (1; 100; 200) comprising:
- main containment means (10) comprising feeding inlet (37) suited to feed said organic substance (O) to be processed into the inner volume (V) defined by said main containment means (10);
- an inlet way (14; 135) suited to receive secondary containment means (12; 112), said secondary containment means (12; 112) being suited to receive a disinfectant substance (S) suited to disinfect said organic substance (O) or suited to receive a cleaning and disinfectant substance (S') suited to disinfect said equipment (1; 100; 200);
- an RFID reader (60) suited to interact with an RFID tag (62; 162) associated with said secondary containment means (12; 112);
- a logic control unit (24);
wherein said method comprises a step of reading at least one piece of information contained in said RFID tag (62; 162) through said RFID reader (60), said piece of information being suited to determine whether said disinfectant substance (S) or said cleaning and disinfectant substance (S') has expired;
**characterized in that** said piece of information comprises the shelf life (Pv) of said disinfectant substance (S) or said cleaning and disinfectant substance (S') from the opening of said secondary containment means (12; 112), wherein the logic control unit (24) establishes the opening date when the secondary containment means (12;112) is inserted into the equipment so that when the RFID reader (60) acquires from the RFID tag (62) said information that the disinfectant substance is **characterized by** a shelf life (Pv) starting from the opening of said secondary containment means (12), the logic control unit (24) will allow the use of the disinfectant substance (S) only within the shelf life.

2. Method according to claim 1), **characterized in that** it comprises a step of inhibiting the feeding of said disinfectant substance (S) or of said cleaning and disinfectant substance (S') into said main containment means (10) if said disinfectant substance (S) or said cleaning and disinfectant substance (S') has expired.

3. Method according to claim 1) or 2), **characterized in that** it comprises a step of generating a signal suited to indicate that said disinfectant substance (S) or said cleaning and disinfectant substance (S') has expired.

4. Method according to any of the preceding claims, **characterized in that** said piece of information comprises the expiry date (Ds) of said disinfectant substance (S) or said cleaning and disinfectant substance (S').

## Patentansprüche

1. Verfahren zum Betreiben einer Anlage (1; 100; 200) zur Verarbeitung einer organischen Substanz (O), wobei die Anlage (1; 100; 200) Folgendes umfasst:
- Hauptbehältermittel (10), umfassend einen Zufuhreinlass (37), der dazu geeignet ist, die zu verarbeitende organische Substanz (O) in das durch die Hauptbehältermittel (10) definierte Innenvolumen (V) einzubringen;
- einen Einlasskanal (14; 135), der dazu geeignet ist, Sekundärbehältermittel (12; 112) aufzunehmen, wobei die Sekundärbehältermittel (12; 112) dazu geeignet sind, eine Desinfektionssubstanz (S) aufzunehmen, die dazu geeignet ist, die organische Substanz (O) zu desinfizieren, oder die dazu geeignet sind, eine Reinigungs- und Desinfektionssubstanz (S') aufzunehmen, die dazu geeignet ist, die Anlage (1; 100; 200) zu desinfizieren;
- ein RFID-Lesegerät (60), das dazu geeignet ist, mit einem RFID-Tag (62; 162) zu interagieren, das mit den Sekundärbehältermitteln (12; 112) verknüpft ist;
- eine Logiksteuereinheit (24),
wobei das Verfahren einen Schritt des Auslesens mindestens einer in dem RFID-Tag (62; 162) enthaltenen Information mittels des RFID-Lesegeräts (60) umfasst, wobei diese Information dazu geeignet ist, festzustellen, ob die Desinfektionssubstanz (S) oder die Reinigungs- und Desinfektionssubstanz (S') abgelaufen ist,
**dadurch gekennzeichnet, dass** die Information die Haltbarkeitsdauer (Pv) der Desinfektionssubstanz (S) oder der Reinigungs- und Desinfektionssubstanz (S') ab dem Öffnen der Sekundärbehältermittel (12; 112) umfasst, wobei die Logiksteuereinheit (24) das Öffnungsdatum ermittelt, wenn die Sekundärbehältermittel (12; 112) in die Anlage eingesetzt werden, sodass die Logiksteuereinheit (24), wenn das RFID-Lesegerät (60) anhand des RFID-Tags (62) die Information erfasst, dass die Desinfektionssubstanz eine Haltbarkeitsdauer (Pv) aufweist, die mit dem Öffnen der Sekundärbehältermittel (12) beginnt, die Verwendung der Desinfektionssubstanz (S) nur innerhalb dieser Haltbarkeitsdauer zulässt.

2. Verfahren nach Anspruch **1), dadurch gekennzeichnet, dass** es einen Schritt des Unterbindens des Einbringens der Desinfektionssubstanz (S) oder der Reinigungs- und Desinfektionssubstanz (S') in die Hauptbehältermittel (10) umfasst, wenn die Desinfektionssubstanz (S) oder die Reinigungs- und Desinfektionssubstanz (S') abgelaufen ist.

3. Verfahren nach Anspruch 1) oder 2), **dadurch gekennzeichnet, dass** es einen Schritt des Generierens eines Signals umfasst, das dazu geeignet ist, anzuzeigen, dass die Desinfektionssubstanz (S) oder die Reinigungs- und Desinfektionssubstanz (S') abgelaufen ist.

4. Verfahren nach einem der vorhergehenden Ansprüche, **dadurch gekennzeichnet, dass** die Information das Verfallsdatum (Ds) der Desinfektionssubstanz (S) oder der Reinigungs- und Desinfektionssubstanz (S') umfasst.

## Revendications

1. Méthode pour le fonctionnement d'un équipement (1 ; 100 ; 200) pour le traitement d'une substance organique (O), ledit équipement (1 ; 100 ; 200) comprenant :
- un moyen de confinement principal (10) comprenant une entrée d'alimentation (37) adaptée pour alimenter ladite substance organique (O) à traiter dans le volume intérieur (V) défini par ledit moyen de confinement principal (10) ;
- une voie d'entrée (14 ; 135) adaptée pour recevoir des moyens de confinement secondaires (12 ; 112), lesdits moyens de confinement secondaires (12 ; 112) étant adaptés pour recevoir une substance désinfectante (S) adaptée pour désinfecter ladite substance organique (O) ou adaptés pour recevoir une substance nettoyante et désinfectante (S') adaptée pour désinfecter ledit équipement (1 ; 100 ; 200) ;
- un lecteur RFID (60) adapté pour interagir avec une étiquette RFID (62 ; 162) associée auxdits moyens de confinement secondaires (12 ; 112) ;
- une unité de commande logique (24) ;
ladite méthode comprenant une étape consistant à lire au moins un élément d'information contenu dans ladite étiquette RFID (62 ; 162) à l'aide dudit lecteur RFID (60), ledit élément d'information étant adapté pour déterminer si ladite substance désinfectante (S) ou ladite substance nettoyante et désinfectante (S') est périmée ;
**caractérisée en ce que** ledit élément d'information comprend la durée de conservation (Pv) de ladite substance désinfectante (S) ou de ladite substance nettoyante et désinfectante (S') à compter de l'ouverture desdits moyens de confinement secondaires (12 ; 112), l'unité de commande logique (24) établissant la date d'ouverture lorsque les moyens de confinement secondaires (12 ; 112) sont insérés dans l'équipement, de sorte que lorsque le lecteur RFID (60) acquiert, à partir de l'étiquette RFID (62), ledit élément d'information indiquant que la substance désinfectante est **caractérisée par** une durée de conservation (Pv) à compter de l'ouverture dudit moyen de confinement secondaire (12), l'unité de commande logique (24) n'autorise l'utilisation de la substance désinfectante (S) que pendant la durée de conservation.

2. Méthode selon la revendication 1), **caractérisée en ce qu'**elle comprend une étape consistant à empêcher l'alimentation de ladite substance désinfectante (S) ou de ladite substance nettoyante et désinfectante (S') dans ledit moyen de confinement principal (10) si ladite substance désinfectante (S) ou ladite substance nettoyante et désinfectante (S') est périmée.

3. Méthode selon la revendication 1) ou 2), **caractérisée en ce qu'**elle comprend une étape consistant à générer un signal adapté pour indiquer que ladite substance désinfectante (S) ou ladite substance nettoyante et désinfectante (S') est périmée.

4. Méthode selon l'une quelconque des revendications précédentes, **caractérisée en ce que** ledit élément d'information comprend la date de péremption (Ds) de ladite substance désinfectante (S) ou de ladite substance nettoyante et désinfectante (S').
